(19) Europäisches Patentamt / European Patent Office / Office européen des brevets

(11) **EP 3 363 366 B1**

(12) **EUROPEAN PATENT SPECIFICATION**

(45) Date of publication and mention of the grant of the patent:
**21.04.2021 Bulletin 2021/16**

(51) Int Cl.:
*A61B 8/02* (2006.01)   *A61B 8/08* (2006.01)
*G01S 15/89* (2006.01)   *A61B 8/00* (2006.01)

(21) Application number: **15906102.7**

(22) Date of filing: **23.10.2015**

(86) International application number:
**PCT/CN2015/092741**

(87) International publication number:
**WO 2017/063220 (20.04.2017 Gazette 2017/16)**

(54) **A DEVICE THAT DETECTS VASCULAR CONDITION BASED ON POINT FLUCTUATION CONDUCTION CHARACTERISTICS OF THE HEART**

VORRICHTUNG ZUM NACHWEIS EINER GEFÄSSERKRANKUNG AUF DER BASIS EINES LEITFÄHIGKEITSSCHWANKUNGSCHARAKTERISTIKUMS AN EINER HERZSTELLE

DISPOSITIF DE DÉTECTION D'UNE AFFECTION VASCULAIRE SUR LA BASE D'UNE CARACTÉRISTIQUE DE FLUCTUATION DE CONDUCTANCE EN UN POINT CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2015 CN 201510658939**

(43) Date of publication of application:
**22.08.2018 Bulletin 2018/34**

(73) Proprietor: **Guangzhou Phonpad Information Technology Cooperation Limited Guangzhou, Guangdong 510630 (CN)**

(72) Inventors:
 • **WEI, Gang**
 **Guangzhou**
 **Guangdong 510663 (CN)**
 • **LIU, Jiaojiao**
 **Guangzhou**
 **Guangdong 510663 (CN)**
 • **WANG, Yige**
 **Guangzhou**
 **Guangdong 510663 (CN)**
 • **CAO, Yan**
 **Guangzhou**
 **Guangdong 510663 (CN)**
 • **YANG, Cui**
 **Guangzhou**
 **Guangdong 510663 (CN)**
 • **MA, Biyun**
 **Guangzhou**
 **Guangdong 510663 (CN)**
 • **LI, Jie**
 **Guangzhou**
 **Guangdong 510663 (CN)**
 • **ZHAO, Mingjian**
 **Guangzhou**
 **Guangdong 510663 (CN)**

(74) Representative: **Puschmann Borchert Kaiser Klettner Patentanwälte Partnerschaft mbB Bajuwarenring 21 82041 Oberhaching (DE)**

(56) References cited:
EP-A1- 1 421 905        WO-A1-2014/197908
WO-A1-2015/056239       CN-A- 1 845 707
CN-A- 101 022 765       CN-U- 202 173 413
US-A1- 2005 004 467     US-A1- 2006 100 530
US-A1- 2007 123 779     US-A1- 2012 116 229
US-A1- 2014 343 423     US-A1- 2015 080 727
US-B1- 6 511 436

• K LOSSIUS ET AL: "Fluctuations in blood flow to acral skin in humans: connection with heart rate and blood pressure variability.", THE JOURNAL OF PHYSIOLOGY, vol. 460, no. 1, 1 January 1993 (1993-01-01), pages 641-655, XP055524622, GB ISSN: 0022-3751, DOI: 10.1113/jphysiol.1993.sp019491

**Description**

The present invention relates generally to pulse wave and vascular condition detection technology. More specifically it relates to a device that detect vascular condition based on point fluctuation conduction characteristics of the heart.

Cardiovascular disease is a common disease that seriously threatens human health. Routine examinations include blood pressure test, electrocardiogram (ECG), complete blood count, blood cholesterol and blood sugar tests, etc. Among them, ECG makes use of electrodes placed on the skin to record the electrical activity of the heart over a period of time. These electrodes detect the tiny electrical changes on the skin that arise from the heart muscle's electrophysiologic pattern of depolarizing and repolarizing during each heartbeat so as to determine the health condition of the heart. ECG is helpful in diagnosing arrhythmias, myocardial ischemia, myocardial infarction, and other heart diseases. However, the human body is a good conductor of electricity. The electrical signal recorded on the skin is the superposition of the electrical signals generated by the heart muscle after passing through multiple paths. Therefore, it is inaccurate to diagnose heart diseases based on the electrical signal measured on the skin.

Pulse diagnosis is a unique diagnosis method of traditional Chinese medicine. Chinese medicine practitioners, by palpating the patient's radial arteries and sensing the depth, speed, strength, rhythm and shape of the arterial pulse, can infer the cause and condition of the disease and determine the prognosis of the disease according to the obtained pulses. However, these pulses are based on practitioners' finger feeling and thus completely depend on the individual's subjective judgments. The arterial pulse at the wrist and its changes form pulse waves, which are waves of blood pushed from the heart into the circulation with each heartbeat. Therefore, the comprehensive information of the shape, strength, speed, rhythm and other aspects of pulse waves reflects many physiological and pathological features of the cardiovascular system. Currently, noninvasive pulse wave detection methods at home and abroad are mainly divided into two categories, the detection method based on pressure waves and that based on volume waves. Physiological information related to cardiovascular, such as heart rate, cardiac output, vascular compliance, and so on, can be obtained through the waveform analysis of pulse waves. For the detection method based on pressure waves, it directly detects pressure waves generated by contraction and expansion of vascular walls of some superficial arteries. Since it has stringent requirements for testing position, it has relatively large interference in the measurement. For the detection method based on volume waves, volume waves are formed when pressure waves are filtered through the resistance of distal capillaries and multiple leaks of the capillary network. In this case, some cardiovascular information might be lost.

By analyzing the characteristics of pulse waves, we can track, diagnose, and predict certain cardiovascular diseases. The existing detection methods are limited to the analysis of the periodic features and changes of pulse waves (without considering the influence of the signal source) and involve less nonlinear distortion information. The pulse wave velocity determined by the time delay estimation is related to not only the elasticity of vessel walls, but also the caliber, thickness, blood density, blood viscosity, and other factors of arterial vessels. Although the existing detection methods can provide an approximate estimation of arterial elasticity, they can determine neither the location of the disease nor the specific cause of the disease (for example, changes in blood viscosity, angiosclerosis, or other reasons).

Targeting at these problems, this patent proposes a device and method that detect vascular condition based on point fluctuation conduction characteristics of the heart. The device directly detects the beats of the heart muscle and vascular walls through active emission of ultrasound and array reception. Then the vascular system between the heart muscle and vascular walls is identified using digital signal processing methods. Since the device has small radiation, simple structure, high detection precision and is easy and safe to operate, it can be widely used in health monitoring, remote examination, auxiliary diagnosis, and other fields.

In US 2006/100530 A1 a device that detects vascular condition based on point fluctuation conduction characteristics of the heart is disclosed. This device comprises a plurality of flexible detection terminals with ultrasonic transceiving capability, a control module; and a computing module. The flexible detection terminal is connected with the control module and the control module is connected with the computing module. The flexible detection terminal is responsible for driving and transmitting the ultrasonic detection signal, receiving and preprocessing the ultrasonic echo signal, and data communications. The control module is responsible for setting and controlling detection parameters of the whole device, generating detection signals, storing detection data, comparing vascular conduction characteristics, data communications, and human-computer interaction. The computing module is responsible for inversion of point fluctuation and pulse waves, identification of vascular conduction characteristics, extraction of vascular features, and data communications.

A similar device is disclosed in WO 2014/197908 A1, US 2007/123779 A1 and EP 1 421 905 A1.

In US 2012/0116229 A1 is disclosed an ultrasound diagnostic apparatus. This apparatus comprises a detector for detecting a direction of radial expansion/contraction of a vascular wall associated with a heartbeat in a long-axis image of the vascular wall based on amplitude information of reception signals outputted from an ultrasound probe when a first ultrasonic beam is transmitted and received to and from a blood vessel of a subject through the ultrasound probe by a transmitter/receiver. Furthermore, a controller for controlling the transmitter/receiver is provided so as to transmit and receive a second ultrasonic beam parallel to the detected vascular wall expansion/contraction direction through the ultrasound probe. Additionally, a tracker for tracking a movement of the vascular wall using amplitude information and phase information of the reception signals outputted from the ultrasound probe is provided when the second ultrasonic beam is transmitted and received. A calculator is for calculating elastic characteristics of the vascular wall based on the tracked vascular wall movement provided.

Heartbeat is the power source of the human blood circulatory system. During systole, the ventricles contract and pump blood through the body. During diastole, the ventricles relax and fill with blood again. This leads to cyclical changes of arterial blood pressure and arterial volume, which result in cyclical fluctuations of the arterial wall and then form arterial pulse waves. Arterial pulse waves can be perceived in some superficial arteries of the human body. Ventricular pressure rises and falls. The fluctuation of the ventricular pressure is conducted to the great vein close to the heart along the inverse direction of the blood flow, which makes the venous wall expand and contract with the fluctuation of the venous pressure and then forms venous pulse waves. Therefore, the heartbeat is the source of pulse waves of the blood vessel, which corresponds to a continuous solution domain having infinite degrees of freedom. According to the finite element method, the detection area of the heart muscle or vascular walls can be discretized into a plurality of detection points. The locations of these detection points are selected according to certain rules and each detection point corresponds to a beat point. Each beat point in the heart detection area can be considered as a point source that forms pulse waves of the blood vessel, i.e., a point wave source. The position of each point wave source changes with time due to heartbeat, which forms a point fluctuation. Due to the expansion and contraction of blood vessels, the position of each beat point in the detection area of the vascular wall also changes, which forms point pulse waves. From the point of view of signal processing, multiple point pulse waves in the detection area of the vascular wall can be regarded as the diffusion function of multiple point fluctuations in the heart detection area after passing through the vascular system. If multiple point fluctuations in the heart detection area are considered as the multiple input signals to the vascular system, then multiple point pulse waves in the detection area of the vascular wall are the multiple output signals of the vascular system. The point fluctuations of the heart muscle and the point pulse waves of the vascular wall can be obtained through active detection using flexible detection terminals placed on the skin. The vascular conduction characteristics between the multi-input signal and the multi-output signal can be identified using digital signal processing methods and then the vascular features can be extracted and compared. In order to achieve the above objective, the present invention provides a device that detects vascular condition based on point fluctuation conduction characteristics of the heart. The device includes a plurality of flexible detection terminals with ultrasonic transceiving capability, a control module and a computing module, wherein the flexible detection terminal is connected with the control module, and the control module is connected with the computing module; the flexible detection terminal is responsible for driving and transmitting the ultrasonic detection signal, receiving and preprocessing the ultrasonic echo signal, and data communications; the control module is responsible for setting and controlling detection parameters of the whole device, generating detection signals, storing detection data, comparing vascular conduction characteristics, data communications, and human-computer interaction; the computing module is responsible for inversion of point fluctuation and pulse waves, identification of vascular conduction characteristics, extraction of vascular features, and data communications.

The above mentioned computing module includes an inversion unit, a conduction characteristic identification unit, a feature extraction unit and a communication unit. Based on the ultrasonic echo signals received by the reception probes of the flexible detection terminal, the inversion unit performs time and spatial time-domain/frequency-domain inversion of the positions and their variations of multiple detection points of the heart muscle and vascular walls in the detection area using frequency estimation algorithms and geometric principles, and obtains multidimensional time-domain/frequency-domain characteristics of multiple point fluctuations of the heart muscle and multiple point pulse waves of the vascular wall. The conduction characteristic identification unit takes the multiple point fluctuations of the heart muscle as the input and the multiple point pulse waves of the vascular wall as the output, identifies the vascular conduction characteristics between the multi-input signal and the multi-output signal using digital signal processing methods, and then decomposes the vascular conduction characteristics based on the cascade feature of the blood vessel to obtain the conduction characteristics of a certain section of the blood vessel. The feature extraction unit is responsible for extracting the features of the vascular conduction characteristics and then obtaining the conduction features of a certain section of the blood vessel. The communication unit is responsible for the data and control information communication between the computing module and the control module using the wired/wireless communication method.

The abovementioned flexible detection terminal includes an ultrasonic transceiver probe array, a transmission processing unit, a reception processing unit, and a communication unit, all of which are fixed on a flexible circuit board and packaged using sound transparent and waterproof material. Flexible detection terminals are fixed by tape on the external skin close to the heart or the vascular wall and firmly against the skin using couplant. In the flexible detection terminal, the ultrasonic transceiver probe array is composed of multiple ultrasonic probes that are arranged according to a certain rule and form an array, wherein one of the probes is responsible for ultrasonic emission and the other probes are responsible for ultrasonic echo reception; the transmission processing unit performs amplification and D/A conversion on the signal received from the control module according to the set parameters and through the transmission probe the obtained electrical signal is converted into an ultrasonic signal, which is then emitted; the reception processing unit performs amplification, A/D conversion and data buffering on the received signal according to the set parameters; the communication unit is responsible for the data and control information communication between the flexible detection terminal and the control module using the wired/wireless communication method.

The abovementioned control module includes a broadband signal generation unit, a master controller, a storage unit, a feature comparison unit, a human-computer interaction unit, a communication unit, and a data buffering unit. The broadband signal generation unit is responsible for generating and driving the broadband detection signal, which is sent to different flexible detection terminals through the communication unit; the master controller is responsible for scheduling among different units to ensure the normal operation of the entire system; the storage unit is in charge of storing the classification feature database, detection locations, vascular conduction features, myocardial point fluctuations, point pulse waves, the patient's symptoms and age, etc.; the feature comparison unit is responsible for carrying out comparison between the vascular conduction characteristics and the feature database; the human-computer interaction unit is responsible for human-computer interaction and can be used for the parameter setting, the input of the control command and the output of the error information; the communication unit is responsible for the data and control information communication between the flexible detection terminal and the control module as well as between the control module and the computing module using the wired/wireless communication method; the data buffering unit is responsible for caching ultrasound echo signals and control parameters.

The above mentioned device for detecting the vascular condition based on point fluctuation conduction characteristics of the heart is configured to perform the following steps:

Step 1: Parameter setting of the device.
The set parameters include: locations of the detection points, parameters of the broadband detection signal, parameters of the inversion unit, parameters of the conduction characteristic identification unit, parameters of the feature extraction unit, parameters of the communication unit, and parameters for displaying detection results;

Step 2: Automatic detection of device status.
Device status detected in this step includes: connection status of the probe array of flexible detection terminals, connection status of the flexible detection terminal and the control module, connection status of the control module and the computing module, connection and online status of the communication unit, power capacity status of flexible detection terminals;

Step 3: Generation of the broadband detection signal.
The broadband signal generation unit of the control module generates the broadband detection signal according to the parameters of the broadband detection signal set in Step 1;

Step 4: Ultrasonic wave transmission.
The communication unit of the flexible detection terminal is responsible for receiving the broadband detection signal generated by the control module. The received signal goes through the amplifier and D/A converter in the transmission processing unit, and is converted by the transmission probe into ultrasonic waves, which are then emitted;

Step 5: Ultrasonic echo reception.
The emitted ultrasonic waves are reflected and form ultrasonic echo when encountering the heart muscle or vascular walls. Part of the ultrasonic echo is received by the adjacent ultrasonic probes and the received echo signal is stored in the data buffering unit after going through the amplifier and A/D converter in the reception processing unit and then sent to the control module through the communication unit;

Step 6: Inversion of point fluctuation/point pulse wave;
Perform time and spatial time-domain/frequency-domain inversion of the positions and their variation of multiple detection points of the heart muscle/vascular walls in the detection area using frequency estimation algorithms and geometric principles, and obtain multidimensional time-domain/frequency-domain characteristics of point fluctuations of the heart muscle or point pulse waves of vascular walls;

Step 7: Identification and decomposition of the conduction characteristics.
Take the multiple point fluctuations of the heart muscle as the input and the multiple point pulse waves of the vascular wall as the output and identify the vascular conduction characteristics between the multi-input signal and the multi-output signal using digital signal processing methods. Then obtain the conduction characteristics of a certain section of the blood vessel by decomposing the vascular conduction characteristics based on the cascade feature of the blood vessel;

Step 8: Feature extraction.

By analyzing the vascular conduction characteristics in the time domain/frequency domain, obtain the time-domain/frequency-domain conduction feature of a certain section of the blood vessel;

Step 9: Result storing and classification.

According to the vascular conduction feature, judge the condition of the blood vessel detected and display it on the human-computer interaction platform. Also store the classification feature database, detection locations, vascular conduction features, myocardial point fluctuations, point pulse waves, the patient's symptoms and age, etc., in the storage unit and the detection results are divided into the following three categories and processed separately.

(1) with disease symptoms. The detection results in this category indicate that a certain section of the blood vessel possesses relevant pathological features and matches the feature information in the classification feature database. The feature information of the vascular conduction characteristics will be retained in the feature database of the corresponding symptom in the storage unit;

(2) no disease symptoms. The detection results in this category indicate that a certain section of the blood vessel does not possess relevant pathological features and the data in the storage unit are directly discarded;

(3) suspicious disease symptoms. The detection results in this category cannot provide a definite diagnosis of the vascular condition. Such feature data are stored in the storage unit and when the detection device is available or the test is completed, the testing staff can choose whether to perform repeated tests and more accurate data processing.

The beneficial effect of the invention is as follows.

(1) In the present invention, the detection area of the heart muscle/vascular walls is discretized into a plurality of detection points. Through continuous active detection of the positions and their variations of the detection points, the point fluctuations of the heart muscle and the point pulse waves of the vascular wall can be obtained. Therefore, detection accuracy is improved through continuous detection and rich information is acquired through multi-point detection.

(2) In this invention, an ultrasonic transceiver probe array is set in the flexible detection terminal. Active emission of ultrasonic waves and continuous array reception of ultrasonic echoes are adopted. Time and spatial multidimensional inversion of point fluctuations/point pulse waves of the detection point is carried out using echo analysis, frequency estimation algorithms and geometric principles. The information carried is rich, which improves detection accuracy.

(3) In this invention, the multiple point fluctuations of the heart muscle are taken as the input and the multiple point pulse waves of the vascular wall are taken as the output. Then the vascular conduction characteristics between the input signal and the output signal can be identified using digital signal processing methods. Therefore, the feature information of the blood vessel is rich and complete, which can improve detection accuracy.

(4) The present invention can provide the conduction characteristics of a certain section of the blood vessel by decomposing the vascular conduction characteristics based on the cascade feature of the blood vessel. Through feature extraction and comparison, the condition of a certain section of the blood vessel is obtained, which is helpful to determination and localization of the disease.

(5) The present invention can record detection locations, vascular features, myocardial point fluctuations, point pulse waves, the patient's symptoms and age, etc each time. It can provide a reference for future diagnosis and evidence of curative effect by establishing a database.

(6) The invention has the advantages of low requirements for testing staff, small radiation, simple structure, high detection precision, being easy and safe to operate, etc. Automatic diagnosis and curative effect tracking can be realized based on the data analysis and feature comparison results of the back end.

Further advantages, features and potential applications of the present invention may be gathered from the description which follows, in conjunction with the embodiments illustrated in the drawings.

Throughout the description, the claims and the drawings, those terms and associated reference signs will be used as are notable from the enclosed list of reference signs. In the drawings is shown

Fig. 1   a system block diagram of the device according to the invention;

Fig. 2   a flexible detection terminal of the device according to the invention;

Fig. 3   a schematic of the detection process with a device according to the invention;

Fig. 4   a schematic of the fixation of the flexible detection terminals on the skin according to one embodiment of the invention;

Fig. 5   a schematic of decomposition of vascular conduction characteristics;

Fig. 6   a workflow diagram of the use of the device according to the invention.

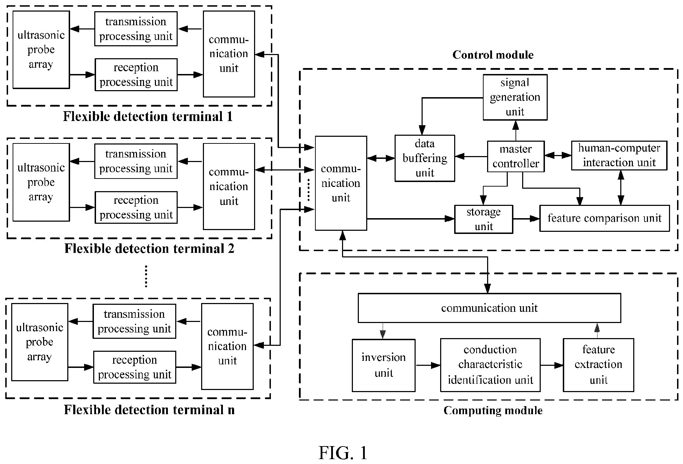

The present invention provides a device that detects vascular condition based on point fluctuation conduction characteristics of the heart, which includes a plurality of flexible detection terminals with ultrasonic transceiving capability, a control module and a computing module. The system block diagram of the invention is shown in FIG. 1. The flexible detection terminal is connected with the control module, and the control module is connected with the computing module; the flexible detection terminal is responsible for driving and transmitting the ultrasonic detection signal, receiving and preprocessing the ultrasonic echo signal, and data communications; the control module is responsible for setting and controlling detection parameters of the whole device, generating detection signals, storing detection data, comparing vascular conduction characteristics, data communications, and human-computer interaction; the computing module is responsible for inversion of point fluctuation and pulse waves, identification of vascular conduction characteristics, extraction of vascular features, and data communications.

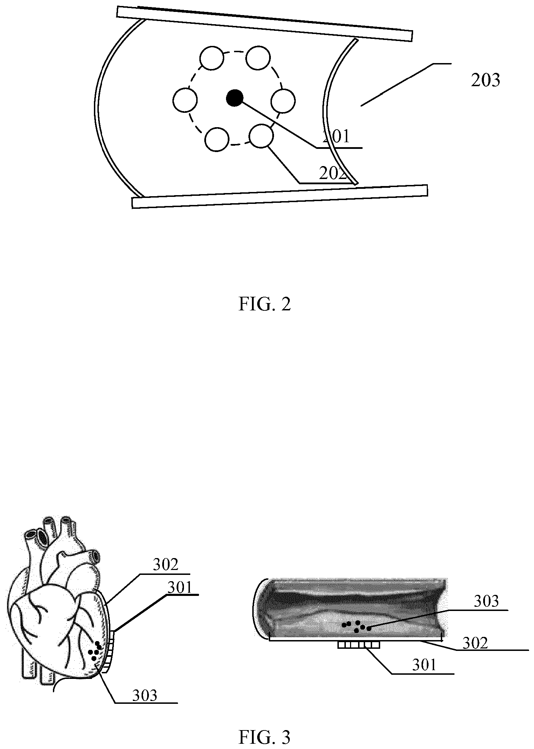

The flexible detection terminal in this embodiment includes an ultrasonic transceiver probe array, a transmission processing unit, a reception processing unit, and a communication unit, all of which are fixed on a flexible circuit board. FIG. 2 illustrates a flexible detection terminal of the invention. The abovementioned ultrasonic transceiver probe array is composed of multiple ultrasonic probes that are arranged according to a certain rule and form an array, wherein the transmission probe 201 in the center is responsible for ultrasonic emission and the reception probes 202 around are responsible for ultrasonic echo reception. The ultrasonic transceiver probe array is fixed on a flexible circuit board 203 and packaged using sound transparent and waterproof material. The detection process of the invention is shown in FIG. 3. During detection, the flexible detection terminal 301 is fixed by tape on 302 the external skin close to the heart and/or the vascular wall and firmly against the skin using couplant. The positions of 303 the selected detection points are inverted through active emission of ultrasonic waves and array reception of ultrasonic echoes. The abovementioned transmission processing unit performs amplification and D/A conversion on the signal received from the control module according to the set parameters and through the transmission probe the obtained electrical signal is converted into an ultrasonic signal, which is then emitted; the abovementioned reception processing unit performs amplification, A/D conversion and data buffering on the received signal according to the set parameters; the abovementioned communication unit is responsible for the data and control information communication between the flexible detection terminal and the control module using the wired/wireless communication method.

The control module in this embodiment includes a broadband signal generation unit, a master controller, a storage unit, a feature comparison unit, a human-computer interaction unit, a communication unit, and a data buffering unit. The abovementioned broadband signal generation unit is responsible for generating and driving the broadband detection signal, which is sent to different flexible detection terminals through the communication unit; the abovementioned master controller is responsible for scheduling among different units to ensure the normal operation of the entire system; the abovementioned storage unit is in charge of storing the classification feature database, detection locations, vascular conduction features, myocardial point fluctuations, point pulse waves, the patient's symptoms and age, etc.; the abovementioned feature comparison unit is responsible for carrying out comparison between the vascular conduction characteristics and the feature database; the abovementioned human-computer interaction unit is responsible for human-computer interaction and can be used for the parameter setting, the input of the control command and the output of the error information; the abovementioned communication unit is responsible for the data and control information communication between the flexible detection terminal and the control module as well as between the control module and the computing module using the wired/wireless communication method; the abovementioned data buffering unit is responsible for caching ultrasound echo signals and control parameters.

The computing module in this embodiment includes an inversion unit, a conduction characteristic identification unit, a feature extraction unit and a communication unit. Based on the ultrasonic echo signals received by the reception probes of the flexible detection terminal, the abovementioned inversion unit performs time and spatial time-domain/frequency-domain inversion of the positions and their variation of multiple detection points of the heart muscle or vascular walls using frequency estimation algorithms and geometric principles, and obtains multidimensional time-domain/frequency-domain characteristics of multiple point fluctuations of the heart muscle or multiple point pulse waves of the vascular wall; the abovementioned conduction characteristic identification unit takes the multiple point fluctuations of the heart muscle as the input and the multiple point pulse waves of the vascular wall as the output, identifies the vascular conduction characteristics between the multi-input signal and the multi-output signal using digital signal processing methods, and then decomposes the vascular conduction characteristics based on the cascade feature of the blood vessel to obtain the conduction characteristics of a certain section of the blood vessel; the abovementioned feature extraction unit is responsible for extracting the feature of the vascular conduction characteristics and then obtaining the conduction feature of a certain section of the blood vessel; the abovementioned communication unit is responsible for the data and control information communication between the computing module and the control module using the wired/wireless communication method.

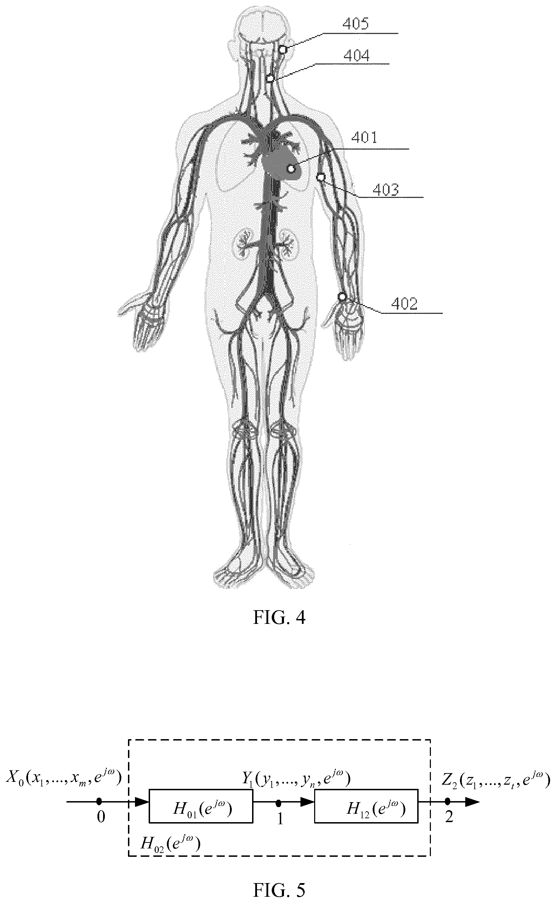

The schematic of this embodiment is shown in FIG. 4. The flexible detection terminals are fixed on the skin that is close to 401 ventricular heart muscle, 402 radial artery, 403 brachial artery, 404 carotid artery, 405 anterior auricular artery, respectively, and cling to the skin using couplant. At these locations, the beats of the heart muscle and the vascular wall are palpable. At each detection location, multiple detection points are selected according to a certain rule. The control module generates broadband detection signals and transmits them to each detection terminal through the communication unit. The signals go through amplification and D/A conversion in the transmission processing unit and through the transmission probe the obtained electrical signals are converted into ultrasonic signals, which are then emitted. Then reflection occurs at the selected detection points on the heart muscle or vascular wall and a part of the reflected waves are received by adjacent ultrasonic receiving probes. The received ultrasonic echoes go through amplification, A/D conversion and data buffering in the reception processing unit. Then the buffered data are sent to the control module through the communication unit. The control module receives the data and after being buffered the data are sent to the computing module. Then the inversion unit performs time and spatial time-domain/frequency-domain inversion of the positions and their variation of multiple detection points of the heart muscle or vascular wall using frequency estimation algorithms and geometric principles, and obtains multiple point fluctuations or multiple point pulse waves. The obtained point fluctuations or multiple point pulse waves are then sent to the conduction characteristic identification unit, which takes the multiple point fluctuations of the heart muscle as the input and the multiple point pulse waves of the vascular wall as the output, and identifies the vascular conduction characteristics between the ventricle and the radial artery, between the ventricle and the brachial artery, between the ventricle and the carotid artery, between the ventricle and the anterior auricular artery, respectively, using digital signal processing methods. Next, based on the cascade feature of the blood vessel, decompose the abovementioned vascular conduction characteristics to obtain the arterial conduction characteristics between the wrist and the upper arm and between the neck and the anterior ear, respectively. In the feature extraction unit, extract the time and frequency feature of the vascular conduction characteristics. The obtained conduction feature is sent to the control module through the communication unit and stored in the storage unit. The master controller compares the received feature information with those in the feature database and diagnoses the vascular condition and disease symptoms, such as angiosteosis, thrombus, angioma, etc. It can also carry out curative effect tracking.

FIG. 5 is a schematic of the decomposition of vascular conduction characteristics of the present invention.

Set a plurality of detection points $x_1,...,x_m$ at detection location 0 of the ventricle. Detected signals from multiple detection points are used as the multi-input signals at this location, i.e., multiple point fluctuation signals, denoted by $X(x_1,...,x_m,e^{j\omega})$ Set a plurality of detection points $y_1,..., y_n$ at detection location 1 of the blood vessel. Detected signals from multiple detection points are used as the multi-output signals at this location, i.e., multiple point pulse waves, denoted by $Y_1(y_1,...,y_n,e^{j\omega})$. Set a plurality of detection points $z_1,...,z_t$ at detection location 2 of the blood vessel. Detected signals from multiple detection points are used as the multi-output signals at this location, i.e., multiple point pulse waves, denoted by $Z_2(z_1,...,z_t,e^{j\omega})$. The vascular conduction characteristics between detection location 0 and detection location 1, between detection location 0 and detection location 2, and between detection location 1 and detection location 2, are denoted by $H_{01}(e^{j\omega})$, $H_{02}(e^{j\omega})$, and $H_{12}(e^{j\omega})$, respectively. Then

$$H_{12}(e^{j\omega}) = \frac{Z_2(z_1,...,z_t,e^{j\omega})}{Y_1(y_1,...,y_n,e^{j\omega})} = \frac{Z_2(z_1,...,z_t,e^{j\omega})/X(x_1,...,x_m,e^{j\omega})}{Y_1(y_1,...,y_n,e^{j\omega})/X(x_1,...,x_m,e^{j\omega})} = \frac{H_{02}(e^{j\omega})}{H_{01}(e^{j\omega})} \quad (1)$$

Therefore, through the detection of myocardial point fluctuations and point pulse waves of the blood vessel, based on the cascade feature of the blood vessel the conduction characteristics of a certain section of the blood vessel can be obtained, which is helpful for localization of vascular disease.

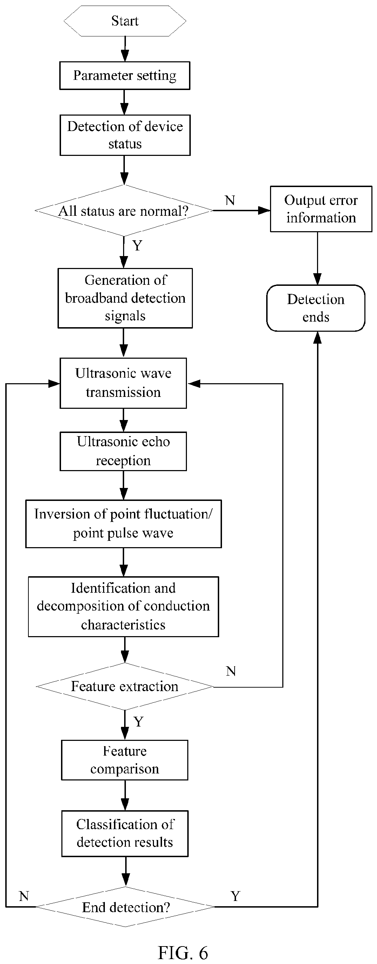

FIG. 6 is a workflow diagram of the invention. The concrete steps are as follows.

Step 1: Parameter setting of the device.

The set parameters include: locations of the detection points, parameters of the broadband detection signal, parameters of the inversion unit, parameters of the conduction characteristic identification unit, parameters of the feature extraction unit, parameters of the communication unit, and parameters for displaying detection results;

Step 2: Automatic detection of device status.

Device status detected in this step includes: connection status of the probe array of flexible detection terminals, connection status of the flexible detection terminal and the control module, connection status of the control module and the computing module, connection and online status of the communication unit, power capacity status of flexible detection terminals;

Step 3: Generation of the broadband detection signal.

The broadband signal generation unit of the control module generates the broadband detection signal according to the parameters of the broadband detection signal set in Step 1;

Step 4: Ultrasonic wave transmission.

The communication unit of the flexible detection terminal is responsible for receiving the broadband detection signal generated by the control module. The received signal goes through the amplifier and D/A converter in the transmission processing unit, and is converted by the transmission probe into ultrasonic waves, which are then emitted;

Step 5: Ultrasonic echo reception.

The emitted ultrasonic waves are reflected and form ultrasonic echo when encountering the heart muscle or vascular walls. Part of the ultrasonic echo is received by the adjacent ultrasonic probes and the received echo signal is stored in the data buffering unit after going through the amplifier and A/D converter in the reception processing unit and then sent to the control module through the communication unit;

Step 6: Inversion of point fluctuation/point pulse wave;

Perform time and spatial time-domain/frequency-domain inversion of the positions and their variation of multiple detection points of the heart muscle/vascular walls in the detection area using frequency estimation algorithms and geometric principles, and obtain multidimensional time-domain/frequency-domain characteristics of point fluctuations of the heart muscle or point pulse waves of vascular walls;

Step 7: Identification and decomposition of the conduction characteristics.

Take the multiple point fluctuations of the heart muscle as the input and the multiple point pulse waves of the vascular wall as the output and identify the vascular conduction characteristics between the multi-input signal and the multi-output signal using digital signal processing methods. Then obtain the conduction characteristics of a certain section of the blood vessel by decomposing the vascular conduction characteristics based on the cascade feature of the blood vessel;

Step 8: Feature extraction.

By analyzing the vascular conduction characteristics in the time domain/frequency domain, obtain the time-domain/frequency-domain conduction feature of a certain section of the blood vessel;

Step 9: Result storing and classification.

According to the vascular conduction feature, judge the condition of the blood vessel detected and display it on the human-computer interaction platform. Also store the classification feature database, detection location, vascular conduction features, myocardial point fluctuations, point pulse waves, the patient's symptoms and age, etc., in the storage unit and the detection results are divided into the following three categories and processed separately.

(1) with disease symptoms. The detection results in this category indicate that a certain section of the blood vessel possesses relevant pathological features and matches the feature information in the classification feature database. The feature information of the vascular conduction characteristics will be retained in the feature database of the corresponding symptom in the storage unit;

(2) no disease symptoms. The detection results in this category indicate that a certain section of the blood vessel does not possess relevant pathological features and the data in the storage unit are directly discarded;

(3) suspicious disease symptoms. The detection results in this category cannot provide a definite diagnosis of the vascular condition. Such feature data are stored in the storage unit and when the detection device is available or the test is completed, the testing staff can choose whether to perform repeated tests and more accurate data processing.

**List of reference signs**

| | |
|---|---|
| 201 | transmission probe |
| 202 | reception probes |
| 203 | flexible circuit board |
| 301 | flexible detection terminal |
| 302 | the external skin close to the heart and/or the vascular wall |
| 303 | the selected detection points |
| 401 | ventricular heart muscle |
| 402 | radial artery |
| 403 | brachial artery |
| 404 | carotid artery |
| 405 | anterior auricular artery |

**Claims**

1. A device that detects vascular condition based on point fluctuation conduction characteristics of the heart, comprising a plurality of flexible detection terminals (301) with ultrasonic transceiving capability;

   a control module; and

   a computing module,

   wherein each flexible detection terminal (301) is connected with the control module;

   wherein the control module is connected with the computing module;

   wherein each flexible detection terminal (301) is configured to drive and transmit an ultrasonic detection signal, receive and preprocessing an ultrasonic echo signal, and perform data communications;

   wherein the control module is configured to set and control detection parameters of the whole device, generate detection signals, store detection data, compare vascular conduction characteristics, perform data communications, and human-computer interaction; and

   wherein the computing module is configured to carry out inversion of point fluctuation and pulse waves, identification of vascular conduction characteristics, extraction of vascular features,

   and data communications, wherein

   the computing module comprises an inversion unit;

   a conduction characteristic identification unit;

   a feature extraction unit; and

   a communication unit,

   wherein based on the ultrasonic echo signals received by the probes responsible for ultrasonic echo reception of the flexible detection terminal (301), the inversion unit is configured to perform time and

   spatial time-domain/frequency-domain inversion of the positions and their variations of multiple detection points (303) of the heart muscle and vascular wall in the detection area using frequency estimation algorithms and geometric principles, and to obtain multidimensional time-domain/frequency-domain characteristics of multiple point fluctuations of the heart muscle and multiple point pulse waves of the vascular wall;

   wherein the conduction characteristic identification unit is configured to take the multiple point fluctuations of the heart muscle as the input and the multiple point pulse waves of the vascular wall as the output, to identify the vascular conduction characteristics between the multi-input signal and the multi-output signal using digital signal processing methods, and then to decompose the vascular conduction characteristics based on the cascade feature of the blood vessel to obtain the conduction characteristics of a certain section of the blood vessel;

wherein the feature extraction unit is configured to extract the feature of the vascular conduction characteristics and then to obtain the conduction feature of a certain section of the blood vessel; and

wherein the communication unit is configured to perform the data and control information communication between the computing module and the control module using a wired/wireless communication method.

2. The device according to claim 1, wherein the flexible detection terminal (301) comprises an ultrasonic transceiver probe array;

a transmission processing unit;

a reception processing unit; and

a communication unit,

wherein all of them are fixed on a flexible circuit board and packaged using sound transparent and waterproof material;

wherein the flexible detection terminals (301) are fixed by tape on the external skin close to the heart and/or the vascular wall (302) and firmly against the skin using couplant;

wherein the ultrasonic transceiver probe array is composed of multiple ultrasonic probes that are arranged according to a certain rule and form an array, wherein one of the probes is responsible for ultrasonic emission and the other probes are responsible for ultrasonic echo reception;

wherein the transmission processing unit is configured to perform amplification and D/A conversion on the signal received from the control module according to the set parameters and through the transmission probe (202) the obtained electrical signal is converted into an ultrasonic signal, which is then emitted;

wherein the reception processing unit is configured to perform amplification, A/D conversion and data buffering on the received signal according to the set parameters; and

wherein the communication unit is configured to perform the data and control information communication between the flexible detection terminal (301) and the control module using a wired/wireless communication method.

3. The device according to claim 1, wherein the control module comprises a broadband signal generation unit;

a master controller;

a storage unit;

a feature comparison unit;

a human-computer interaction unit;

a communication unit; and

a data buffering unit,

wherein the broadband signal generation unit is configured to generate and drive the broadband detection signal, which is sent to different flexible detection terminals (301) through the communication unit;

wherein the master controller is configured to schedule among different units to ensure the normal operation of the entire system;

wherein the storage unit is configured to store the classification feature database, detection locations, vascular conduction features, myocardial point fluctuations, point pulse waves, the patient's symptoms and age, etc.;

wherein the feature comparison unit is configured to carry out comparison between the vascular conduction characteristics and the feature database;

wherein the human-computer interaction unit is configured to perform human-computer interaction and to be used for the parameter setting, the input of the control command and the output of the error information;

wherein the communication unit is configured to perform the data and control information communication between the flexible detection terminal (301) and the control module as well as between the control module and the computing module using the wired/wireless communication method; and

wherein the data buffering unit is configured to cache ultrasound echo signals and control parameters.

4. The device according to claims 2 and 3, configured to perform the following steps:

step 1: parameter setting of the device, wherein the control module is configured to set parameters including locations of the detection points (303), parameters of the broadband detection signal, parameters of the inversion unit, parameters of the conduction characteristic identification unit, parameters of the feature extraction unit, parameters of the communication unit, and parameters for displaying detection results;

step 2: automatic detection of device status, wherein

device status detected in this step includes: connection status of the probe array of flexible detection terminals (301), connection status of the flexible detection terminal (301) and the control module, connection status of the control module and the computing module, connection and online status of the communication unit, power capacity status of flexible detection terminals (301);

step 3: generation of the broadband detection signal, wherein

the broadband signal generation unit of the control module is configured to generate the broadband detection signal according to the parameters of the broadband detection signal set in Step 1;

step 4: ultrasonic wave transmission, wherein

the communication unit of the flexible detection terminal (301) is configured to receive the broadband detection signal generated by the control module; and

wherein the received signal goes through the amplifier and D/A converter in the transmission processing unit, and is converted by the transmission probe into ultrasonic waves, which are then emitted;

step 5: ultrasonic echo reception, wherein

the emitted ultrasonic waves are reflected and form ultrasonic echo when encountering the heart muscle or vascular walls; and

wherein part of the ultrasonic echo is received by the adjacent ultrasonic probes and the received echo signal is stored in the data buffering unit after going through the amplifier and A/D converter in the reception processing unit and then sent to the control module through the communication unit;

step 6: inversion of point fluctuation/point pulse wave, wherein the computing module is configured to perform time and spatial time-domain/frequency-domain inversion of the positions and their variation of multiple detection points of the heart muscle/vascular walls in the detection area using frequency estimation algorithms and geometric principles, and obtain multidimensional time-domain/frequency-domain characteristics of point fluctuations of the heart muscle or point pulse waves of vascular walls;

step 7: identification and decomposition of the conduction characteristics, wherein the computing module is configured to take the multiple point fluctuations of the heart muscle as the input and the multiple point pulse waves of the vascular wall as the output and identify the vascular conduction characteristics between the multi-input signal and the multi-output signal using digital signal processing methods; and further to obtain the conduction characteristics of a certain section of the blood vessel by decomposing the vascular conduction characteristics based on the cascade feature of the blood vessel;

Step 8: feature extraction, wherein

the time-domain/frequency-domain conduction feature of a certain section of the blood vessel is obtained by analyzing the vascular conduction characteristics in the time domain/frequency domain;

step 9: result storing and classification, wherein the control module is configured to judge according to the vascular conduction feature, the condition of the blood vessel detected and display it on the human-computer interaction unit; store the classification feature database, detection locations, vascular conduction features, myocardial point fluctuations, point pulse waves, the patient's symptoms and age, etc., in the storage unit; and wherein the detection results are divided into the following three categories and processed separately:

(1) with disease symptoms, wherein

the detection results in this category indicate that a certain section of the blood vessel possesses relevant pathological features and matches the feature information in the classification feature database; and wherein the feature information of the vascular conduction characteristics will be retained in the feature database of the corresponding symptom in the storage unit;

(2) no disease symptoms, wherein

the detection results in this category indicate that a certain section of the blood vessel does not possess relevant pathological features and the data in the storage unit are directly discarded;

(3) suspicious disease symptoms, wherein

the detection results in this category cannot provide a definite diagnosis of the vascular condition; and wherein such feature data are stored in the storage unit.

**Patentansprüche**

1. Vorrichtung zum Nachweis einer Gefäßerkrankung anhand von punktuellen Schwankungen in den Leitfähigkeitseigenschaften des Herzens, umfassend

eine Vielzahl von flexiblen Detektionsklemmen (301) mit Ultraschall-Sende-/Empfangs-Fähigkeit;

ein Steuermodul; und

ein Rechenmodul,

wobei jede flexible Detektionsklemme (301) mit dem Steuermodul verbunden ist;

wobei das Steuermodul mit dem Rechenmodul verbunden ist;

wobei jede flexible Detektionsklemme (301) zum Ansteuern und Übertragen eines Ultraschalldetektionssignals, zum Empfangen und Vorverarbeiten eines Ultraschallechosignals und zum Durchführen von Datenkommunikation konfiguriert ist;

wobei das Steuermodul zum Einstellen und Steuern von Detektionsparametern der gesamten Vorrichtung, zum Erzeugen von Detektionssignalen, zum Speichern von Detektionsdaten, zum Vergleichen von Gefäßleitfähigkeitseigenschaften, zum Durchführen von Datenkommunikation und Mensch-Computer-Interaktion konfiguriert ist; und wobei das Rechenmodul zum Durchführen einer Inversion von punktuellen Schwankungen und Pulswellen, zum Bestimmen von Gefäßleitfähigkeitseigenschaften, zur Extraktion von Gefäßmerkmalen und zur Datenkommunikation konfiguriert ist, wobei

das Rechenmodul folgendes umfasst:

eine Inversionseinheit;
eine Leitfähigkeitseigenschafts-Bestimmungseinheit;
eine Merkmalsextraktionseinheit; und
eine Kommunikationseinheit,

wobei die Inversionseinheit konfiguriert ist, anhand der von den für den Ultraschallechoempfang der flexiblen Detektionsklemme (301) zuständigen Sonden empfangenen Ultraschallechosignale eine zeitliche und räumliche Zeit-/Frequenzbereichs-Inversion der Positionen und ihrer Variationen von mehreren Detektionspunkten (303) des Herzmuskels und der Gefäßwand im Detektionsbereich unter Verwendung von Frequenzschätzalgorithmen und geometrischen Prinzipien durchzuführen und mehrdimensionale Zeit-/Frequenzbereichs-Charakteristika mehrerer punktueller Schwankungen des Herzmuskels und mehrerer Pulswellen der Gefäßwand zu erhalten;

wobei die Leitfähigkeitseigenschafts-Bestimmungseinheit konfiguriert ist, die Mehrpunkt-Schwankungen des Herzmuskels als Eingang und die Mehrpunkt-Pulswellen der Gefäßwand als Ausgang zu verwenden, die Gefäßleitfähigkeitseigenschaften zwischen dem Mehrfach-Eingangssignal und dem Mehrfach-Ausgangssignal unter Verwendung digitaler Signalverarbeitungsverfahren zu bestimmen und dann die Gefäßleitfähigkeitseigenschaften basierend auf der Kaskadenfunktion des Blutgefäßes zu zerlegen, um die Leitfähigkeitseigenschaften eines bestimmten Abschnitts des Blutgefäßes zu erhalten;

wobei die Merkmalsextraktionseinheit konfiguriert ist, das Merkmal der Gefäßleitfähigkeitseigenschaften zu extrahieren und dann die Leitfähigkeitseigenschaft eines bestimmten Abschnitts des Blutgefäßes zu erhalten; und

wobei die Kommunikationseinheit konfiguriert ist, die Daten- und Steuerinformationskommunikation zwischen dem Rechenmodul und dem Steuermodul unter Verwendung eines drahtgebundenen/drahtlosen Kommunikationsverfahrens durchzuführen.

2. Vorrichtung nach Anspruch 1, bei der die flexible Detektionsklemme (301) folgendes umfasst:

ein Ultraschall-Transceiver-Sonden-Array;
eine Übertragungsverarbeitungseinheit;
eine Empfangsverarbeitungseinheit; und
eine Kommunikationseinheit,

wobei alle diese Komponenten auf einer flexiblen Leiterplatte fixiert und mit schalldurchlässigem und wasserdichtem Material verpackt sind;

wobei die flexiblen Detektionsklemmen (301) mittels Klebeband und unter Verwendung von Koppelmittel in der Nähe des Herzens und/oder der Gefäßwand (302) fest an der Haut anliegend auf der äußeren Haut fixiert werden;

wobei das Ultraschall-Transceiver-Sonden-Array aus mehreren Ultraschallsonden besteht, die entsprechend einer bestimmten Regel angeordnet sind und ein Array bilden, wobei eine der Sonden für die Ultraschallabstrahlung und die anderen Sonden für den Ultraschallechoempfang verwendet werden;

wobei die Übertragungsverarbeitungseinheit konfiguriert ist, eine Verstärkung und eine D/A-Wandlung des vom Steuermodul empfangenen Signals gemäß den eingestellten Parametern durchzuführen und das erhaltene elektrische Signal dann von der Übertragungssonde (202) in ein Ultraschallsignal umgewandelt wird, das dann ausgegeben wird;

wobei die Empfangsverarbeitungseinheit konfiguriert ist, eine Verstärkung, eine A/D-Wandlung und eine Datenpufferung des Empfangssignals gemäß den eingestellten Parametern durchzuführen; und

wobei die Kommunikationseinheit konfiguriert ist, die Daten- und Steuerinformationskommunikation zwischen der flexiblen Detektionsklemme (301) und dem Steuermodul unter Verwendung eines drahtgebundenen/drahtlosen Kommunikationsverfahrens durchzuführen.

3. Vorrichtung nach Anspruch 1, bei der das Steuermodul folgendes umfasst:

eine Einheit zur Erzeugung von Breitbandsignalen;

eine Master-Steuerung;

eine Speichereinheit;

eine Merkmalsvergleichseinheit;

eine Mensch-Computer-Interaktionseinheit

eine Kommunikationseinheit; und

eine Datenpuffereinheit,

wobei die Einheit zur Erzeugung von Breitbandsignalen konfiguriert ist, das Breitbanddetektionssignals zu erzeugen und zu treiben, das dann über die Kommunikationseinheit an verschiedene flexible Detektionsklemmen (301) gesendet wird;

wobei die Master-Steuerung konfiguriert ist, die Zeitsteuerung für die verschiedenen Einheiten vorzunehmen, um den normalen Betrieb des gesamten Systems sicherzustellen;

wobei die Speichereinheit konfiguriert ist, die Klassifizierungsmerkmalsdatenbank, Detektionsorte, Gefäßleitfähigkeitseigenschaften, punktuelle Myokardschwankungen, Punktpulswellen, die Symptome und das Alter des Patienten usw. zu speichern;

wobei die Merkmalsvergleichseinheit konfiguriert ist, einen Vergleich zwischen den Gefäßleitfähigkeitseigenschaften und der Merkmalsdatenbank durchzuführen;

wobei die Mensch-Computer-Interaktionseinheit konfiguriert ist, eine Mensch-Computer-Interaktion durchzuführen und zur Parametereinstellung, zur Eingabe des Steuerbefehls und zur Ausgabe der Fehlerinformation verwendet zu werden;

wobei die Kommunikationseinheit konfiguriert ist, die Daten- und Steuerinformationskommunikation zwischen der flexiblen Detektionsklemme (301) und dem Steuermodul sowie zwischen dem Steuermodul und dem Rechenmodul unter Verwendung des drahtgebundenen/drahtlosen Kommunikationsverfahrens durchzuführen; und

wobei die Datenpuffereinheit für das Zwischenspeichern von Ultraschallechosignalen und Steuerparametern konfiguriert ist.

4. Vorrichtung nach Anspruch 2 und 3, konfiguriert zum Durchführen der folgenden Schritte:

Schritt 1: Einstellen der Parameter der Vorrichtung, wobei

das Steuermodul konfiguriert ist, Parameter einzustellen, einschließlich Orte der Detektionspunkte (303), Parameter des Breitbanddetektionssignals, Parameter der Inversionseinheit, Parameter der Leitfähigkeitsmerkmals-Identifikationseinheit, Parameter der Merkmalsextraktionseinheit, Parameter der Kommunikationseinheit und Parameter zur Anzeige von Detektionsergebnissen;

Schritt 2: automatisches Erfassen des Gerätestatus, wobei

der in diesem Schritt erfasste Gerätestatus folgendes umfasst: Verbindungsstatus des Sonden-Arrays flexibler Detektionsklemmen (301), Verbindungsstatus der flexiblen Detektionsklemme (301) und des Steuermoduls, Verbindungsstatus des Steuermoduls und des Rechenmoduls, Verbindungs- und Online-Status der Kommunikationseinheit, Energiekapazitätsstatus der flexiblen Detektionsklemmen (301);

Schritt 3: Erzeugen des Breitbanddetektionssignals, wobei

die Einheit zur Erzeugung eines Breitbandsignals des Steuermoduls das Breitbanddetektionssignal gemäß den in Schritt 1 eingestellten Parametern des Breitbanddetektionssignals erzeugt;

Schritt 4: Übertragen von Ultraschallwellen, wobei

die Kommunikationseinheit der flexiblen Detektionsklemme (301) konfiguriert ist, das von dem Steuermodul erzeugte Breitbanddetektionssignal zu empfangen; und

wobei das Empfangssignal den Verstärker und den D/A-Wandler in der Übertragungsverarbeitungseinheit durchläuft und von der Übertragungssonde in Ultraschallwellen umgewandelt wird, die dann ausgesendet werden;

Schritt 5: Ultraschallecho-Empfang, wobei

die ausgesendeten Ultraschallwellen beim Auftreffen auf den Herzmuskel oder auf Gefäßwände reflektiert werden und dabei ein Ultraschallecho erzeugen; und

wobei ein Teil des Ultraschallechos von den benachbarten Ultraschallsonden empfangen wird und das empfangene Echosignal in der Datenpuffereinheit gespeichert wird, nachdem es den Verstärker und den A/D-Wandler in der Empfangsverarbeitungseinheit durchlaufen hat, und dann durch die Kommunikationseinheit an das Steuermodul gesendet wird;

Schritt 6: Invertieren der Punktschwankung/Punktpulswelle, wobei

das Rechenmodul konfiguriert ist, eine zeitliche und räumliche Zeit-/Frequenzbereichs-Inversion der Positionen und ihrer Variationen von mehreren Detektionspunkten des Herzmuskels/der Gefäßwände im Detektionsbereich unter Verwendung von Frequenzschätzalgorithmen und geometrischen Prinzipien durchzuführen, und mehrdimensionale Zeit-/Frequenzbereichs-Charakteristiken von Punktschwankungen des Herzmuskels oder Punktpulswellen von Gefäßwänden zu erhalten;

Schritt 7: Identifizieren und Zerlegen der Leitfähigkeitseigenschaften, wobei

das Rechenmodul konfiguriert ist, die Mehrpunkt-Schwankungen des Herzmuskels als Eingang und die Mehrpunkt-Pulswellen der Gefäßwand als Ausgang zu verwenden und die Gefäßleitfähigkeitseigenschaften zwischen dem Mehrfach-Eingangssignal und dem Mehrfach-Ausgangssignal unter Verwendung digitaler Signalverarbeitungsverfahren zu bestimmen; und des Weiteren die Leitfähigkeitseigenschaften eines bestimmten Abschnitts des Blutgefäßes zu erhalten, indem die Gefäßleitfähigkeitseigenschaften basierend auf der Kaskadenfunktion des Blutgefäßes zerlegt werden;

Schritt 8: Merkmalsextraktion, wobei

das Zeit-/Frequenzbereichs-Leitfähigkeitsmerkmal eines bestimmten Abschnitts des Blutgefäßes durch Analysieren der Gefäßleitfähigkeitseigenschaften im Zeit-/Frequenzbereich erhalten wird;

Schritt 9: Speichern und Klassifizieren der Ergebnisse, wobei

das Steuermodul konfiguriert ist, entsprechend dem Gefäßleitfähigkeitsmerkmal den Zustand des detektierten Blutgefäßes zu beurteilen und auf der Mensch-Computer-Interaktionseinheit anzuzeigen;

die Klassifizierungsmerkmalsdatenbank, Erfassungsorte, Gefäßleitfähigkeitseigenschaften, myokardiale Punktschwankungen, Punktpulswellen, die Symptome und das Alter des Patienten usw. in der Speichereinheit zu speichern; und

wobei die Detektionsergebnisse in die folgenden drei Kategorien unterteilt und getrennt verarbeitet werden:

(1) mit Krankheitssymptomen, wobei

die Detektionsergebnisse in dieser Kategorie anzeigen, dass ein bestimmter Abschnitt des Blutgefäßes relevante pathologische Merkmale aufweist und mit den Merkmalsinformationen in der Klassifikationsmerkmalsdatenbank übereinstimmt; und

wobei die Merkmalsinformation der Gefäßleitfähigkeitseigenschaften in der Merkmalsdatenbank des entsprechenden Symptoms in der Speichereinheit beibehalten wird;

(2) ohne Krankheitssymptome, wobei

die Detektionsergebnisse in dieser Kategorie anzeigen, dass ein bestimmter Abschnitt des Blutgefäßes keine relevanten pathologischen Merkmale besitzt, und die Daten in der Speichereinheit direkt verworfen werden;

(3) verdächtige Krankheitssymptome, wobei

anhand der Detektionsergebnisse in dieser Kategorie keine eindeutige Diagnose der Gefäßerkrankung möglich ist; und

wobei diese Merkmalsdaten in der Speichereinheit gespeichert werden.

## Revendications

1. Dispositif qui détecte un état vasculaire, sur la base de caractéristiques de conduction de fluctuation en un point du cœur, comprenant :

une pluralité de bornes de détection flexibles (301) ayant une capacité d'émission-réception d'ultrasons ;
un module de commande ; et
un module de calcul,
dans lequel chaque borne de détection flexible (301) est connectée au module de commande ;
dans lequel le module de commande est connecté au module de calcul ;
dans lequel chaque borne de détection flexible (301) est conçue pour acheminer et transmettre un signal de détection à ultrasons, recevoir et prétraiter un signal d'écho ultrasonore, et assurer des communications de données ;
dans lequel le module de commande est conçu pour régler et commander des paramètres de détection de l'ensemble du dispositif, produire des signaux de détection, stocker des données de détection, comparer des caractéristiques de conduction vasculaire, assurer des communications de données et une interaction homme-ordinateur ; et
dans lequel le module de calcul est conçu pour effectuer une inversion de fluctuation de point et d'ondes de pouls, une identification de caractéristiques de conduction vasculaire, une extraction de particularités vasculaires, et des communications de données, dans lequel :
le module de calcul comprend :

une unité d'inversion ;

une unité d'identification de caractéristiques de conduction ;

une unité d'extraction de particularités ; et

une unité de communication,

dans lequel, sur la base des signaux d'écho ultrasonore reçus par les sondes chargées de la réception d'écho ultrasonore de la borne de détection flexible (301), l'unité d'inversion est conçue pour réaliser une inversion temporelle et spatiale dans le domaine temporel/domaine fréquentiel des positions et de leurs variations de multiples points de détection (303) du muscle cardiaque et de la paroi vasculaire dans la zone de détection, à l'aide d'algorithmes d'estimation de fréquence et de principes géométriques, et pour obtenir des caractéristiques multidimensionnelles dans le domaine temporel/domaine fréquentiel des fluctuations de multiples points du muscle cardiaque et des ondes de pouls de multiples points de la paroi vasculaire ;

dans lequel l'unité d'identification de caractéristiques de conduction est conçue pour prendre les fluctuations de multiples points du muscle cardiaque comme entrée et les ondes de pouls de multiples points de la paroi vasculaire comme sortie, pour identifier les caractéristiques de conduction vasculaire entre le signal à entrées multiples et le signal à sorties multiples à l'aide de procédés de traitement de signaux numériques, puis pour décomposer les caractéristiques de conduction vasculaire sur la base des particularités en cascade du vaisseau sanguin afin d'obtenir les caractéristiques de conduction d'un certain segment du vaisseau sanguin ;

dans lequel l'unité d'extraction de particularités est conçue pour extraire la particularité des caractéristiques de conduction vasculaire, puis pour obtenir la particularité de conduction d'un certain segment du vaisseau sanguin ; et

dans lequel l'unité de communication est conçue pour assurer la communication de données et d'informations de commande entre le module de calcul et le module de commande à l'aide d'un procédé de communication filaire/sans fil.

2. Dispositif selon la revendication 1, dans lequel la borne de détection flexible (301) comprend :

un réseau de sondes ultrasonores émettrices-réceptrices ;

une unité de traitement de transmission ;

une unité de traitement de réception ;

une unité de communication,

dans lequel la totalité d'entre eux est fixée sur une carte de circuit imprimé flexible et encapsulée à l'aide d'un matériau transparent aux sons et imperméable à l'eau ;

dans lequel les bornes de détection flexibles (301) sont fixées par ruban adhésif sur la peau extérieure à proximité du cœur et/ou de la paroi vasculaire (302) et fermement contre la peau à l'aide d'un couplant;

dans lequel le réseau de sondes ultrasonores émettrices-réceptrices se compose de multiples sondes ultrasonores qui sont agencées selon une certaine règle et forment un réseau, dans lequel l'une des sondes est chargée de l'émission d'ultrasons et les autres sondes sont chargées de la réception de l'écho ultrasonore ;

dans lequel l'unité de traitement de transmission est conçue pour réaliser une amplification et une conversion N/A sur le signal reçu en provenance du module de commande, en fonction des paramètres réglés et par le biais de la sonde de transmission (202), le signal électrique obtenu est converti en un signal ultrasonore qui est ensuite émis ;

dans lequel l'unité de traitement de réception est conçue pour réaliser une amplification, une conversion A/N et une mise en mémoire tampon de données sur le signal reçu, en fonction des paramètres réglés ; et

dans lequel l'unité de communication est conçue pour assurer la communication de données et d'informations de commande entre la borne de détection flexible (301) et le module de commande à l'aide d'un procédé de communication filaire/sans fil.

3. Dispositif selon la revendication 1, dans lequel le module de commande comprend :

une unité de production de signal à large bande ;

un contrôleur central ;

une unité de stockage ;

une unité de comparaison de particularités ;

une unité d'interaction homme-ordinateur ;

une unité de communication ; et

une unité de mise en mémoire tampon de données,

dans lequel l'unité de production de signal à large bande est conçue pour produire et acheminer le signal de détection à large bande qui est envoyé à différentes bornes de détection flexibles (301) par le biais de l'unité de communication ;

dans lequel le contrôleur central est conçu pour assurer l'ordonnancement entre différentes unités afin de garantir le fonctionnement normal de l'ensemble du système ;

dans lequel l'unité de stockage est conçue pour stocker la base de données de particularités de classification, les emplacements de détection, les particularités de conduction vasculaire, les fluctuations de points myocardiques, les ondes de pouls de points, les symptômes et l'âge du patient, etc. ;

dans lequel l'unité de comparaison de particularités est conçue pour établir une comparaison entre les caractéristiques de conduction vasculaire et la base de données de particularités ;

dans lequel l'unité d'interaction homme-ordinateur est conçue pour assurer l'interaction homme-ordinateur et pour permettre le réglage de paramètres, l'entrée de l'instruction de commande et la sortie des informations d'erreurs ;

dans lequel l'unité de communication est conçue pour assurer la communication de données et d'informations de commande entre la borne de détection flexible (301) et le module de commande, ainsi qu'entre le module de commande et le module de calcul, à l'aide du procédé de communication filaire/sans fil ; et

dans lequel l'unité de mise en mémoire tampon de données est conçue pour mettre en cache des signaux d'écho ultrasonore et des paramètres de commande.

4. Dispositif selon les revendications 2 et 3, conçu pour réaliser les étapes suivantes :

étape 1 : le réglage de paramètres du dispositif, dans lequel :
le module de commande est conçu pour régler des paramètres incluant les emplacements des points de détection (303), les paramètres du signal de détection à large bande, les paramètres de l'unité d'inversion, les paramètres de l'unité d'identification de caractéristiques de conduction, les paramètres de l'unité d'extraction de particularités, les paramètres de l'unité de communication et les paramètres destinés à l'affichage des résultats de détection ;
étape 2 : la détection automatique de l'état du dispositif, dans lequel :
l'état du dispositif, détecté à cette étape, inclut : l'état de connexion du réseau de sondes des bornes de détection flexibles (301), l'état de connexion de la borne de détection flexible (301) et du module de commande, l'état de connexion du module de commande et du module de calcul, l'état de connexion et en ligne de l'unité de communication, l'état de capacité de puissance des bornes de détection flexibles (301) ;
étape 3 : la production du signal de détection à large bande, dans lequel :
l'unité de production de signal à large bande du module de commande est conçue pour produire le signal de détection à large bande, en fonction des paramètres du signal de détection à large bande, réglés à l'étape 1 ;
étape 4 : la transmission d'ondes ultrasonores, dans lequel :

l'unité de communication de la borne de détection flexible (301) est conçue pour recevoir le signal de détection à large bande produit par le module de commande ; et
dans lequel le signal reçu passe par l'amplificateur et le convertisseur N/A dans l'unité de traitement de transmission, et est converti par la sonde de transmission en ondes ultrasonores qui sont ensuite émises ;

étape 5 : la réception d'écho ultrasonore, dans lequel :

les ondes ultrasonores émises sont réfléchies et forment un écho ultrasonore lorsqu'elles rencontrent le muscle cardiaque ou des parois vasculaires ; et
dans lequel une partie de l'écho ultrasonore est reçue par les sondes ultrasonores adjacentes et le signal d'écho reçu est stocké dans l'unité de mise en mémoire tampon après être passé par l'amplificateur et le convertisseur A/N dans l'unité de traitement de réception, puis est envoyé au module de commande par le biais de l'unité de communication ;

étape 6 : l'inversion de fluctuation de point/onde de pouls de point, dans lequel :
le module de calcul est conçu pour réaliser une inversion temporelle et spatiale dans le domaine temporel/domaine fréquentiel des positions et de leurs variations de multiples points de détection du muscle cardiaque/des parois vasculaires dans la zone de détection, à l'aide d'algorithmes d'estimation de fréquence et de principes géométriques, et pour obtenir des caractéristiques multidimensionnelles dans le domaine temporel/domaine fréquentiel de fluctuations de points du muscle cardiaque ou d'ondes de pouls de points des parois vasculaires ;
étape 7 : l'identification et la décomposition des caractéristiques de conduction, dans lequel le module de calcul est conçu pour :

prendre les fluctuations de multiples points du muscle cardiaque comme entrée et les ondes de pouls de multiples points de la paroi vasculaire comme sortie et identifier les caractéristiques de conduction vasculaire entre le signal à entrées multiples et le signal à sorties multiples à l'aide de procédés de traitement de signaux numériques ; et

en outre pour :

obtenir les caractéristiques de conduction d'un certain segment du vaisseau sanguin en décomposant les caractéristiques de conduction vasculaire sur la base des particularités en cascade du vaisseau sanguin ;

étape 8 : l'extraction de particularités, dans lequel :

la particularité de conduction dans le domaine temporel/domaine fréquentiel d'un certain segment du vaisseau sanguin est obtenue par analyse des caractéristiques de conduction vasculaire dans le domaine temporel/domaine fréquentiel ;

étape 9 : le stockage et la classification de résultats, dans lequel :

le module de commande est conçu pour :

déterminer, en fonction de la particularité de conduction vasculaire, l'état du vaisseau sanguin soumis à détection et l'afficher sur l'unité d'interaction homme-ordinateur ;

stocker la base de données de particularités de classification, les emplacements de détection, les particularités de conduction vasculaire, les fluctuations de points myocardiques, les ondes de pouls de points, les symptômes et l'âge du patient, etc., dans l'unité de stockage ; et dans lequel les résultats de détection sont répartis dans les trois catégories suivantes et traités séparément :

(1) avec des symptômes de maladie, dans lequel :

les résultats de détection de cette catégorie indiquent qu'un certain segment du vaisseau sanguin présente des particularités pathologiques pertinentes et concorde avec les informations de particularités dans la base de données de particularités de classification ; et dans lequel les informations de particularités des caractéristiques de conduction vasculaire seront conservées dans la base de données de particularités du symptôme correspondant dans l'unité de stockage ;

(2) sans symptômes de maladie, dans lequel :

les résultats de détection de cette catégorie indiquent qu'un certain segment du vaisseau sanguin ne présente pas de particularités pathologiques pertinentes et les données dans l'unité de stockage sont directement rejetées ;

(3) avec des symptômes de suspicion de maladie, dans lequel :

les résultats de détection de cette catégorie ne peuvent pas fournir un diagnostic formel de l'état vasculaire ; et

dans lequel de telles données de particularités sont stockées dans l'unité de stockage.

FIG. 1

203

201

202

FIG. 2

302

301

303

303

302

301

FIG. 3

FIG. 4

$$X_0(x_1,...,x_m,e^{j\omega}) \quad Y_1(y_1,...,y_n,e^{j\omega}) \quad Z_2(z_1,...,z_t,e^{j\omega})$$

$$H_{01}(e^{j\omega}) \qquad H_{12}(e^{j\omega})$$

$$H_{02}(e^{j\omega})$$

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006100530 A1
- WO 2014197908 A1
- US 2007123779 A1
- EP 1421905 A1
- US 20120116229 A1